# FASCICULE DE BREVET EUROPEEN

(11) **EP 3 036 218 B1**
(45) Date de publication et mention de la délivrance du brevet: **10.04.2019**
(21) Numéro de dépôt: 14749937.0
(22) Date de dépôt: 30.07.2014
(51) Int. Cl.: C07C 303/32, C07C 233/18

(54) **CO-CRISTAUX D'AGOMELATINE ET D'ACIDE P-TOLUENESULFONIQUE, LEUR PROCEDE DE PREPARATION ET LES COMPOSITIONS PHARMACEUTIQUES QUI LES CONTIENNENT**
CO-KRISTALLE VON AGOMELATIN UND P-TOLUOLSULFONSÄURE, VERFAHREN ZU DEREN HERSTELLUNG UND PHARMAZEUTISCHE ZUSAMMENSETZUNGEN, DIE DIESE ENTHALTEN
CO-CRYSTALS OF AGOMELATINE AND P-TOLUENESULPHONIC ACID, METHOD FOR PREPARING SAME AND THE PHARMACEUTICAL COMPOSITIONS CONTAINING SAME

(30) Priorité: 31.07.2013 WO PCT/CN2013/080472; 17.10.2013 FR 1360121
(43) Date de publication de la demande: 29.06.2016
(73) Titulaire: Les Laboratoires Servier, 92284 Suresnes (FR); Shanghai Institute Of Pharmaceutical Industry, Shanghai 200040 (CN)
(72) Inventeur: SHAN, Hanbin, Gaoan Jiangsu 330800 Jiangxi Province (CN); SHEN, Yuhui, Shanghai 200439 (CN); LUO, Ying, Nanchang City Jiangxi Province, Jiangxi 300006 (CN); LETELLIER, Philippe, F-45000 Orléans (FR); LYNCH, Michael, F-45140 Saint Jean de la Ruelle (FR)
(74) Mandataire: Bestel, Delphine
(86) Numéro de dépôt international: PCT/FR2014/051972
(87) Numéro de publication internationale: WO 2015/015118

(56) Documents cités:
- CN-A- 102 702 041

## Description

La présente invention concerne de nouvelles formes de co-cristaux d'agomélatine et d'acide *p*-toluènesulfonique, leur procédé de préparation ainsi que les compositions pharmaceutiques qui les contiennent.

L'agomélatine ou *N*-[2-(7-methoxy-1-naphtyl)ethyl]acétamide a la structure de formule (II) :

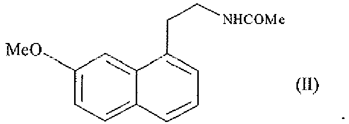

L'agomélatine est commercialisé sous le nom de marque Valdoxan® ou Thymanax® par le groupe français Servier en tant qu'agoniste sur les récepteurs du système mélatoninergique et antagoniste du récepteur 5-HT_{2C}. C'est le premier antidépresseur de type mélatoninergique, utile dans le traitement de la dépression majeure, améliorant le sommeil et la fonction sexuelle.

L'agomélatine, sa préparation et son utilisation en thérapeutique ont été décrits dans les brevets européens EP 0 447 285 et EP1564202.

Compte-tenu de son intérêt pharmaceutique, il est important de pouvoir produire l'agomélatine ou un de ses complexes avec une pureté, une solubilité et une reproductibilité améliorées.

Un procédé de préparation du co-cristal agomélatine/acide *p*-toluènesulfonique a été reporté dans la demande de brevet CN102702041, dans laquelle la structure du co-cristal a été identifiée par ¹HNMR, le produit obtenu étant amorphe.

La forme amorphe présente un certain nombre d'inconvénients en tant que produit pharmaceutique tels qu'une adhérence aux parois, de mauvaises propriétés d'écoulement, une faible stabilité, ainsi il est toujours intéressant de pouvoir disposer d'une forme cristalline bien définie d'une entité chimique.

L'objet de la présente invention consiste en l'élaboration de nouvelles formes cristallines de co-cristaux d'agomélatine et d'acide *p*-toluènesulfonique, présentant d'excellentes propriétés en matière de solubilité, stabilité et pureté, permettant d'envisager leur utilisation pour la fabrication de compositions pharmaceutiques contenant de l'agomélatine.

La présente invention concerne des formes cristallines de co-cristaux d'agomélatine et d'acide *p*-toluènesulfonique ayant la structure de formule (I) : 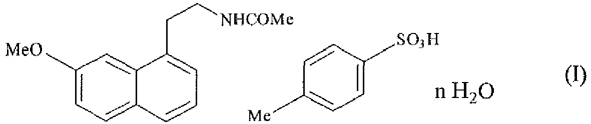 dans laquelle n représente 0 ou 1.

Les composés préférés selon l'invention sont les co-cristaux d'agomélatine et d'acide p-toluènesulfonique suivants :
- co-cristal agamelatine/acide *p*-toluènesulfonique (1/1) monohydrate,
- co-cristal agomelatine/acide *p*-toluènesulfonique (1/1).

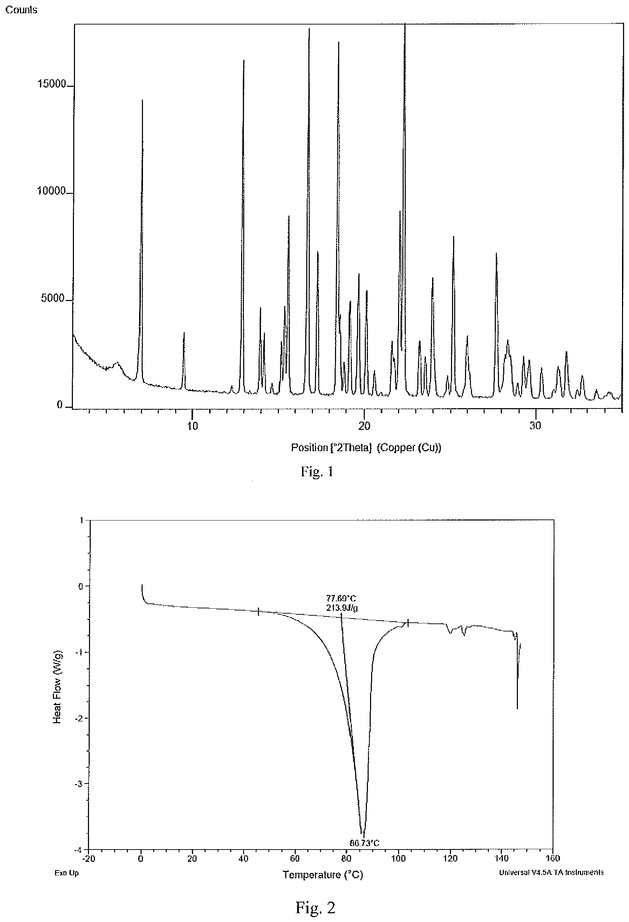

Le co-cristal agomelatine/acide *p*-toluènesulfonique (1/1) monohydrate est caractérisé par son diagramme de diffraction X sur poudre rprésenté dans la Figure 1, mesuré sur un diffractomètre Panalytical Xpert Pro MPD (anticathode de cuivre). Les raies principales sont exprimées en termes de distance inter-réticulaire d, d'angle de Bragg 2 thêta (exprimés en °±0,2), et d'intensité relative (exprimée en pourcentage par rapport à la raie la plus intense) et sont listées dans le Tableau 1:

**Tableau 1: Tableau des pics de diffraction du co-cristal agomelatine/acide p-toluènesulfonique (1/1) monohydrate**

| 2-Theta (°) exp. | d (Å) exp. | Intensité (%) |
|---|---|---|
| 6,9631 | 12,6846 | 77,52 |
| 9,4831 | 9,31871 | 16,21 |
| 12,8823 | 6,86648 | 100 |
| 13,9527 | 6,34201 | 23,16 |
| 14,1761 | 6,24258 | 16,06 |
| 15,1817 | 5,83128 | 13,38 |
| 15,379 | 5,75689 | 25 |
| 15,5788 | 5,68351 | 46,24 |
| 16,7156 | 5,29947 | 94,46 |
| 17,2926 | 5,12391 | 37,98 |
| 18,4671 | 4,80058 | 92,47 |
| 18,6356 | 4,75756 | 22,34 |
| 19,199 | 4,6192 | 25,69 |
| 19,6747 | 4,50857 | 35,74 |
| 20,1398 | 4,4055 | 28,53 |
| 21,6248 | 4,1062 | 14,93 |
| 22,0586 | 4,02643 | 52,23 |
| 22,2859 | 3,98587 | 99,09 |
| 23,2175 | 3,82799 | 15,22 |
| 23,9607 | 3,71092 | 32,37 |
| 25,1733 | 3,53485 | 42,09 |
| 26,0152 | 3,42233 | 16,64 |
| 27,7148 | 3,2162 | 39,29 |
| 28,23 | 3,15866 | 11,25 |
| 28,4033 | 3,13979 | 16,33 |

Lorsque le co-cristal de la présente invention est caractérisé par la mesure de diffraction aux rayons X, il peut y avoir des erreurs de mesure des pics identifiés parfois attribuables à l'équipement ou aux conditions utilisées. Plus particulièrement, les valeurs 2 thêta peuvent avoir une erreur d'environ ± 0,2, et parfois une erreur d'environ ± 0,1 même si des équipements de haute technicité sont utilisés. Ainsi, l'erreur de mesure doit être prise en compte lors de l'identification de la structure du co-cristal.

La structure cristalline du co-cristal agomelatine/acide *p*-toluènesulfonique (1/1) monohydrate a été déterminée et les paramètres suivants ont été identifiés:
- Groupe d'espace : P 21 21 21 (19)
- Paramètres de maille: a = 13,7359(3)Å, b = 25,3716(6)Å, c = 6,4487(1)Å; α = 90°, β = 90(2)°, γ = 90°
- Volume de la maille : V_{unit cell} = 2247,4Å³

Le co-cristal agomelatine/acide *p*-toluènesulfonique (1/1) monohydrate est également caractérisé par DSC (differential scanning calorimetry) dans le spectre représenté dans la Figure 2, qui montre un large endotherme correspondant à la déshydratation du co-cristal et sa fusion à une température d'environ 78°C (et un pic de température à environ 87°C).

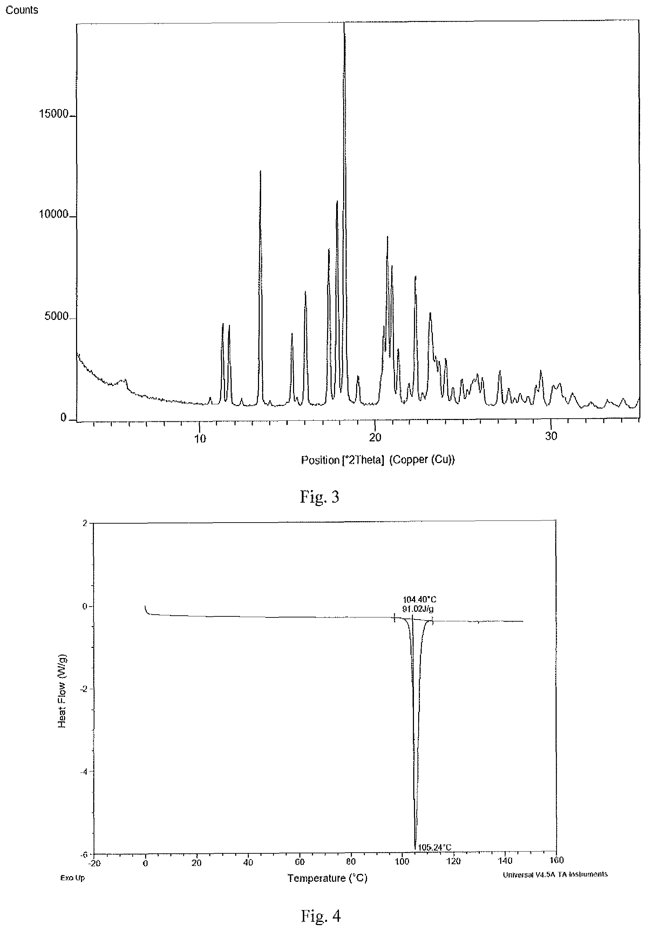

L'invention concerne également le co-cristal agomélatine/acide *p*-toluènesulfonique (1/1) qui est caractérisé par son diagramme de diffraction X sur poudre représenté dans la Figure 3, mesuré sur un diffractomètre Panalytical Xpert Pro MPD (anticathode de cuivre). Les raies principales sont exprimées en termes de distance inter-réticulaire d, d'angle de Bragg 2 thêta (exprimés en °±0,2), et d'intensité relative (exprimée en pourcentage par rapport à la raie la plus intense) et sont listées dans le Tableau 2:

**Tableau 2: Tableau des pics de diffraction du co-cristal agomélatine/acide p-toluènesulfonique (1/1)**

| 2-Theta (°) exp. | d (Å) exp. | Intensité (%) |
|---|---|---|
| 11,2964 | 7,82664 | 21,53 |
| 11,6596 | 7,58367 | 20,45 |
| 13,4436 | 6,58103 | 61,31 |
| 15,2416 | 5,80848 | 18,42 |
| 16,0185 | 5,52847 | 30,89 |
| 17,3473 | 5,10789 | 41,39 |
| 17,8289 | 4,97096 | 54,3 |
| 18,2535 | 4,85629 | 100 |
| 20,4891 | 4,33118 | 19,84 |
| 20,6912 | 4,28932 | 45,12 |
| 20,9516 | 4,23659 | 36,73 |
| 21,3088 | 4,16638 | 14,93 |
| 22,2998 | 3,98342 | 33,92 |
| 23,129 | 3,84244 | 24,66 |
| 23,4107 | 3,79685 | 12,89 |
| 23,6474 | 3,75938 | 12,34 |
| 23,9983 | 3,7052 | 12,8 |

Lorsque le co-cristal de la présente invention est caractérisé par la mesure de diffraction aux rayons X, il peut y avoir des erreurs de mesure des pics identifiés parfois attribuables à l'équipement ou aux conditions utilisées. Plus particulièrement, les valeurs 2 thêta peuvent avoir une erreur d'environ ± 0,2, et parfois une erreur d'environ ± 0,1 même si des équipements de haute technicité sont utilisés. Ainsi, l'erreur de mesure doit être prise en compte lors de l'identification de la structure du co-cristal.

La structure cristalline du co-cristal agomélatine/acide *p*-toluènesulfonique (1/1) a été déterminée et les paramètres suivants ont été identifiés:
- Groupe d'espace : P 2₁2₁2₁ (19)
- Paramètres de maille: a = 8.6683(3) Å, b = 30.360(1) Å, c = 8.0982(4) Å; α = 90°, β = 90°, γ = 90°
- Volume de la maille : V_{unit cell} = 2131.2 Å³

Le co-cristal agomélatine/acide *p*-toluènesulfonique (1/1) est également caractérisé par DSC (differential scanning calorimetry) dans le spectre représenté dans la Figure 4, qui montre un endotherme correspondant à la fusion du complexe à une température d'environ 105°C.

L'invention concerne également un procédé d'obtention des co-cristaux d'agomélatine et d'acide *p*-toluènesulfonique, dans lequel :
- l'agomélatine et l'acide *p*-toluènesulfonique monohydrate sont mélangés au sein d'un solvant organique ou hydro-organique dans les proportions désirées;
- la solution obtenue est agitée et optionnellement chauffée à une température au plus égale à la température d'ébullition du solvant choisi ;
- le milieu est refroidi sous agitation et le complexe précipite naturellement ou précipite après reprise dans un deuxième solvant ;
- le précipité obtenu est filtré et séché ;
- optionnellement, le précipité est séché en chauffant.

Dans le procédé selon l'invention, le solvant utilisé est préférentiellement un éther comme par exemple l'éther diisopropylique, le tétrahydrofurane, le dioxane ou l'éther de méthyle et de *tert*-butyle ; ou un hydrocarbure aromatique comme le toluène par exemple. Lorsqu'un deuxième solvant est utilisé pour favoriser la précipitation du complexe, le solvant choisi est un alcool comme par exemple le méthanol, l'éthanol ou le *tert*-butanol ; un alkane comme par exemple le n-hexane ou le n-heptane ; ou le benzonitrile.

Un procédé alternatif consiste à co-broyer les deux constituants du co-cristal. Préférentiellement le co-broyage est effectué dans une jarre en acier. Une variante de ce procédé consiste à rajouter un solvant organique lors du broyage ; dans ce cas le co-cristal obtenu est ensuite séché. Parmi les solvants utilisés on citera plus particulièrement les éthers comme par exemple l'éther diisopropylique, ou l'éther de méthyle et de *tert*-butyle. Les alcools comme par exemple le méthanol ou le tert-butanol peuvent également être utilisés.

Avantageusement, le broyage est réalisé avec des billes en inox. Le broyage est réalisé au moyen de vibrations, préférentiellement des vibrations avec une fréquence allant de 20 à 30 Hz. Les vibrations sont appliquées pendant une durée pouvant aller de 5 minutes à 3 heures.

Un autre procédé alternatif consiste à mélanger deux solutions contenant chacun des constituants et à congeler rapidement à très basse température le mélange obtenu, puis à sécher à cette même basse température le co-cristal ainsi obtenu. Avantageusement, les deux constituants sont mélangés au sein d'un solvant organique ou hydro-organique. De façon préférée, la congélation et le séchage sont effectués entre -40°C et -60°C, et plus préférentiellement à -40°C.

Un autre procédé avantageux selon l'invention consiste à mélanger les poudres d'agomélatine et de l'acide considéré dans un mélangeur, puis à l'extruder par extrusion bi-vis sans filière pour obtenir un grain solide directement en sortie d'extrudeuse. De préférence, le profil de vis utilisé est un profil à fort cisaillement, avec optionnellement l'utilisation d'éléments malaxeurs permettant d'améliorer la surface de contact entre les constituants. Le paramètre L/D de la vis peut varier entre 10 et 40 et la vitesse de rotation entre 10 et 200 tr/min. La température utilisée varie de 40 à 100 °C.

Les co-cristaux d'agomélatine et d'acide *p*-toluènesulfonique obtenus ont une solubilité accrue de façon très significative par rapport à l'agomélatine *per se,* ce qui les rend plus appropriés pour l'élaboration de formulations pharmaceutiques. Les co-cristaux d'agomélatine et d'acide *p*-toluènesulfonique selon l'invention présentent de plus des propriétés avantageuses de stabilité, pureté et solubilité. Ils sont par ailleurs obtenus par un procédé simple, ne comportant aucune étape difficile.

L'études pharmacologique des co-cristaux selon l'invention montre qu'ils peuvent être utilisés pour le traitement des désordres du système mélatoninergique, et plus particulièrement dans le traitement du stress, des troubles du sommeil, des troubles de l'anxiété et notamment du trouble anxiété généralisée, des troubles obsessionnels compulsifs, des troubles de l'humeur et notamment des troubles bipolaires, de la dépression majeure, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, de la douleur, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, les co-cristaux selon l'invention pourront être utilisés dans les dysfonctionnements sexuels, en tant qu'inhibiteurs de l'ovulation, immunomodulateurs et dans le traitement des cancers.

L'invention s'étend aussi aux compositions pharmaceutiques renfermant comme principe actif un co-cristal d'agomélatine et d'acide *p*-toluènesulfonique selon l'invention avec un ou plusieurs adjuvants ou excipients.

Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les granulés, les comprimés sublinguaux, les gélules, les tablettes, les suppositoires, les crèmes, les pommades, les gels dermiques, les préparations injectables, les suspensions buvables et les pâtes à mâcher.

La posologie utile est adaptable selon la nature et la sévérité de l'affection, la voie d'administration ainsi que l'âge et le poids du patient. Cette posologie varie de 0,1 mg à 1 g par jour d'agomélatine en une ou plusieurs prises.

Des exemples représentatifs de la présente invention sont illustrés avec les Figures correspondantes afin de mieux en apprécier l'objet, les caractéristiques, et les avantages.
Figure 1 : diagramme de diffraction X sur poudre du co-cristal agomelatine/acide p-toluènesulfonique (1/1) monohydrate.
Figure 2 : thermogramme DSC du co-cristal agomelatine/acide *p*-toluènesulfonique (1/1) monohydrate.
Figure 3 : diagramme de diffraction X sur poudre du co-cristal agomelatine/acide *p-*toluènesulfonique (1/1).
Figure 4 : thermogramme DSC du co-cristal agomelatine/acide *p*-toluènesulfonique (1/1).

### Exemple 1 : Co-cristal agomelatine/acide p-toluènesulfonique (1/1) monohydrate

### Mode-opératoire 1

L'agomelatine (5,00g, 1 eq) et l'acide *p*-toluènesulfonique monohydrate (3,92g, 1 eq) sont placés dans un réacteur. 40ml de tétrahydrofurane et 20 ml d'hexane sont ajoutés. La suspension est agitée sous reflux pendant 0,5 heure jusqu'à ce qu'elle devienne limpide (si elle ne devient pas limpide, on rajoute du tétrahydrofurane jusqu'à ce qu'elle le soit). La solution est refroidie naturellement jusqu'à 5°C et agitée 0,5 h, puis la suspension est filtrée. Le gâteau est séché pendant 1 heure sous vide. 8,53g d'un solide blanc sont obtenus.
Rendement: 95,8%
Point de fusion : 78°C

### Mode-opératoire 2

Agomelatine (5,00g, 1 eq) et l'acide *p*-toluènesulfonique monohydrate (3,952g, 1 eq) sont introduits dans un réacteur. 40 mL d'acétone et 10 mL d'hexane sont ajoutés. La suspension est agitée sous reflux pendant 0,5 heure jusqu'à ce qu'elle devienne limpide (si elle ne devient pas limpide, on rajoute de l'acétone jusqu'à ce qu'elle le soit). La solution est refroidie naturellement jusqu'à 5°C et agitée 0,5 h, puis la suspension est filtrée. Le gâteau est séché pendant 1 heure sous vide. 8,06g d'un solide blanc sont obtenus.
Rendement : 90.4%
Point de fusion : 78°C

### Mode-opératoire 3

L'agomelatine (0,5g) et l'acide *p*-toluènesulfonique monohydrate (0,392) sont placés dans une jarre de 50ml non oxydable. Deux billes en acier inox de 12 mm de diamètre sont ajoutées et la jarre est fermée. Des vibrations ayant une fréquence de 30 Hz sont appliquées pendant 15 minutes pour conduire, après une nuit de séchage à température ambiante, à 0,881g de solide.
Point de fusion : 78°C

### Mode-opératoire 4

L'agomelatine (0,5g) et l'acide *p*-toluènesulfonique monohydrate (0,392) sont placés dans une jarre de 50ml non oxydable. Deux billes en acier inox de 12 mm de diamètre sont ajoutées et la jarre est fermée. 100µl d'éther de méthyle et de *tert*-butyle sont ajoutés. Des vibrations ayant une fréquence de 30 Hz sont appliquées pendant 30 minutes pour conduire, après une nuit de séchage à température ambiante, à 0,883g de solide.
Point de fusion : 78°C

### Mode-opératoire 5

L'agomelatine (5g) et l'acide *p*-toluènesulfonique monohydrate (3,92g) sont placés dans une jarre de 100ml non oxydable. Deux billes en acier inox de 12 mm de diamètre sont ajoutées et la jarre est fermée. 100µl d'éther de méthyle et de *tert*-butyle sont ajoutés. Des vibrations ayant une fréquence de 30 Hz sont appliquées pendant 30 minutes pour conduire, après une nuit de séchage à température ambiante, à 8,83g de solide.
Point de fusion : 78°C

### Exemple 2 : Co-cristal agomelatine/acide p-toluènesulfonique (1/1)

2g du co-cristal agomelatine/acide *p*-toluènesulfonique (1/1) monohydrate obtenu dans l'Exemple 1 sont chauffés à 85°C pendant 4 heures. Un solide blanc est obtenu.
Rendement: 100%
Point de fusion : 105°C

Dans les exemples ci-dessus, on peut utiliser l'agomélatine disponible dans le commerce ou préparé selon une des méthodes décrites dans l'art antérieur.

### Exemple 3 : Compositions pharmaceutiques : gélules dosées à 25 mg d'agomélatine

| Formulation pour la préparation de 1000 gélules contenant chacune 25 mg d'agomélatine | |
|---|---|
| Composé de l'Exemple 1 | 44,5 g |
| Lactose (Spherolac 100) | 85,2 g |
| Amidon 1500 | 25,5 g |
| CMS-Na | 8,5 g |
| Ac-Di-Sol ® (FMC) | 17 g |
| Acide Stéarique | 3,4 g |

| Formulation pour la préparation de 1000 gélules contenant chacune 25 mg d'agomélatine | |
|---|---|
| Composé de l'Exemple 2 | 42,7 g |
| Lactose (Spherolac 100) | 85,2 g |
| Amidon 1500 | 25,5 g |
| CMS-Na | 8,5 g |
| Ac-Di-Sol ® (FMC) | 17 g |
| Acide Stéarique | 3,4 g |

### Exemple 4 : Compositions pharmaceutiques : comprimés dosés à 25 mg d'agomélatine

Formulation pour la préparation de 1000 comprimés contenant chacun 25 mg d'agomélatine:

| | |
|---|---|
| Composé de l'Exemple 1 | 44,5 g |
| Lactose monohydrate | 115 g |
| Stéarate de Magnésium | 2 g |
| Amidon de maïs | 33 g |
| Maltodextrines | 15 g |
| Silice colloïdale anhydre | 1 g |
| Amidon de maïs prégélatinisé, Type A | 9 g |

Formulation pour la préparation de 1000 comprimés contenant chacun 25 mg d'agomélatine:

| | |
|---|---|
| Composé de l'Exemple 2 | 42,7 g |
| Lactose monohydrate | 115 g |
| Stéarate de Magnésium | 2 g |
| Amidon de maïs | 33 g |
| Maltodextrins | 15 g |
| Silice colloïdale anhydre | 1 g |
| Amidon de maïs prégélatinisé, Type A | 9 g |

### Méthodes de détection et Résultats

### 1. Pureté des échantillons

Conditions chromatographiques : colonne C18 ; phase mobile : tampon phosphate 10mmol/L (ajusté à pH 7,0 avec NaOH) : acétonitrile 2 :7 (v/v) ; température de la colonne : 40°C ; longueur d'onde de détection : 220 nm ; méthode standard interne utilisée avec le composé des Exemples 1 et 2.

Des solutions à 1 mg/ml des composés de l'invention sont préparées avec la phase mobile. 10 µl de chaque solution sont injectés dans le système de chromatographie liquide et les chromatogrammes sont enregistrés.

Les composés des Exemples 1 et 2 ont des puretés supérieures ou égales à 99%.

### 2. Stabilité

Des échantillons des composés des Exemples 1 et 2 sont placés dans des incubateurs à 40°C pendant 30 jours afin de déterminer leur stabilité par HPLC. Les résultats sont présentés dans le tableau 3 :

**Tableau 3**

| | 25°C, 60%HR BO | 40°C, 75%HR BO | 50°C BF | 70°C BF |
|---|---|---|---|---|
| Composé de l'Exemple 1 | Stable | Stable | Stable | Stable |
| Composé de l'Exemple 2 | Se transforme en monohydrate | Se transforme en monohydrate | Se transforme en monohydrate | Stable |

| | | | | |
|---|---|---|---|---|
| HR: Humidité Relative; BO: Bouteille Ouverte; BF: Bouteille Fermée | | | | |

### 3. Solubilité dans l'eau

A l'aide d'une méthode standard externe, les composés des Exemples 1 et 2 sont testés en HPLC, et comparés avec l'agomélatine de forme II. Les résultats sont présentés dans le tableau 4 sous forme de % d'accroissement de la solubilité par rapport à la solubilité de l'agomélatine de forme II :

**Tableau 4**

| Echantillon | Solubilité (accroissement versus Agomélatine forme II) | | |
|---|---|---|---|
| | Dans l'eau | Dans HCl 0,1N | Dans un tampon pH6,8 |
| Composé de l'Exemple 1 | +41% | +50% | +49% |
| Composé de l'Exemple 2 | +35% | +45% | +60% |

Les résultats montrent que les co-cristaux d'agomélatine et d'acide *p*-toluènesulfonique de la présente invention ont une solubilité supérieure à l'agomélatine de forme II *per se* dans l'eau, dans HCl 0,1N, semblable aux fluides gastriques humains, ou dans un tampon à pH6,8. Ces résultats montrent que les co-cristaux ont un bien meilleur potentiel en terme de biodisponibilité que l'agomélatine de forme II.

### 4. Analyses DSC

Environ 5-10 mg des composés des Exemples 1 et 2 sont pesés dans un creuset en aluminium fermé avec un couvercle en aluminium percé (non hermétique), sauf précision contraire. L'échantillon est introduit dans un appareil TA Q1000 (équipé avec un refroidisseur), refroidi et maintenu à 25°C. Après stabilisation thermique, l'échantillon et la référence sont chauffés de 200°C à 250°C à une vitesse de 10°C/min et la réponse au flux thermique est enregistrée. L'azote est utilisé comme gaz de purge, à un débit de 100 cm³/min.

Les thermogrammes de DSC obtenus avec les composés des Exemples 1 et 2 sont rapportés dans les Figures 2 et 4.

### 5. Analyse de la structure cristalline

Les conditions de mesure des diagrammes de diffraction X sur poudre des produits des Exemples 1 et 2 sont les suivantes :
Environ 50mg des composés des Exemples 1 et 2 sont placés entre deux films Kapton® et fixés sur le support d'échantillons. L'échantillon est ensuite placé dans un diffractomètre PANALYTICAL XPERT-PRO MPD en mode transmission dans les conditions suivantes :
Paramètres du générateur: 45 kV / 40 mA,
Configuration theta/theta
Anode : Cu
K-Alphal [Å] 1,54060
K-Alpha2 [Å] 1,54443
K-Beta [Å] 1,39225
K-A2 / K-A1 Ratio 0,50000
Mode de balayage : continu de 3° à 55° (angle de Bragg 2 thêta)
Pas [°2Th.] 0,0170
Durée du pas [s] 35,5301
Angle de départ [°2Th.] 3,0034
Angle de fin [°2Th.] 54,9894
Rotation: oui

Les diagrammes de diffraction X sur poudre obtenus pour les Exemples 1 et 2 sont représentés dans les Figures 1 et 3.

## Revendications

1. Formes cristallines de co-cristaux d'agomélatine et d'acide *p*-toluènesulfonique de formule (I) : 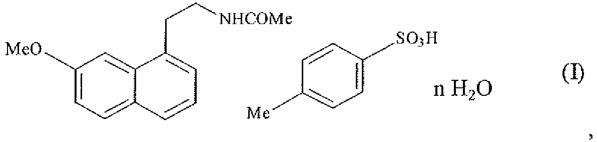 dans laquelle n représente 0 ou 1.

2. Co-cristal d'agomélatine et d'acide *p*-toluènesulfonique monohydrate de formule (I) selon la revendication 1 **caractérisé par** son diagramme de diffraction X sur poudre exprimé en termes de distance inter-réticulaire d, d'angle de Bragg 2 thêta (exprimés en °±0,2), et d'intensité relative suivant:
| 2-Theta (°) exp. | d (Å) exp. | Intensity (%) |
|---|---|---|
| 6,9631 | 12,6846 | 77,52 |
| 9,4831 | 9,31871 | 16,21 |
| 12,8823 | 6,86648 | 100 |
| 13,9527 | 6,34201 | 23,16 |
| 14,1761 | 6,24258 | 16,06 |
| 15,1817 | 5,83128 | 13,38 |
| 15,379 | 5,75689 | 25 |
| 15,5788 | 5,68351 | 46,24 |
| 16,7156 | 5,29947 | 94,46 |
| 17,2926 | 5,12391 | 37,98 |
| 18,4671 | 4,80058 | 92,47 |
| 18,6356 | 4,75756 | 22,34 |
| 19,199 | 4,6192 | 25,69 |
| 19,6747 | 4,50857 | 35,74 |
| 20,1398 | 4,4055 | 28,53 |
| 21,6248 | 4,1062 | 14,93 |
| 22,0586 | 4,02643 | 52,23 |
| 22,2859 | 3,98587 | 99,09 |
| 23,2175 | 3,82799 | 15,22 |
| 23,9607 | 3,71092 | 32,37 |
| 25,1733 | 3,53485 | 42,09 |
| 26,0152 | 3,42233 | 16,64 |
| 27,7148 | 3,2162 | 39,29 |
| 28,23 | 3,15866 | 11,25 |
| 28,4033 | 3,13979 | 16,33 |
incluant les formes dont les angles de diffraction correspondent à ±0.2° près.

3. Co-cristal d'agomélatine et d'acide *p*-toluènesulfonique de formule (I) selon la revendication 1 **caractérisé par** son diagramme de diffraction X sur poudre exprimé en termes de distance inter-réticulaire d, d'angle de Bragg 2 thêta (exprimés en °±0,2), et d'intensité relative suivant:
| 2-Theta (°) exp. | d (Å) exp. | Intensity (%) |
|---|---|---|
| 11,2964 | 7,82664 | 21,53 |
| 11,6596 | 7,58367 | 20,45 |
| 13,4436 | 6,58103 | 61,31 |
| 15,2416 | 5,80848 | 18,42 |
| 16,0185 | 5,52847 | 30,89 |
| 17,3473 | 5,10789 | 41,39 |
| 17,8289 | 4,97096 | 54,3 |
| 18,2535 | 4,85629 | 100 |
| 20,4891 | 4,33118 | 19,84 |
| 20,6912 | 4,28932 | 45,12 |
| 20,9516 | 4,23659 | 36,73 |
| 21,3088 | 4,16638 | 14,93 |
| 22,2998 | 3,98342 | 33,92 |
| 23,129 | 3,84244 | 24,66 |
| 23,4107 | 3,79685 | 12,89 |
| 23,6474 | 3,75938 | 12,34 |
| 23,9983 | 3,7052 | 12,8 |
incluant les formes dont les angles de diffraction correspondent à ±0.2° près.

4. Procédé d'obtention des co-cristaux d'agomélatine et d'acide *p*-toluènesulfonique selon l'une des revendications 1 à 3 **caractérisé en ce que** :
- l'agomélatine et l'acide *p*-toluènesulfonique monohydrate sont mélangés au sein d'un solvant organique ou hydro-organique dans les proportions désirées;
- la solution obtenue est agitée et optionnellement chauffée à une température au plus égale à la température d'ébullition du solvant choisi ;
- le milieu est refroidi sous agitation et le complexe précipite naturellement ou précipite après reprise dans un deuxième solvant ;
- le précipité obtenu est filtré et séché ;
- optionnellement, le précipité est séché en chauffant.

5. Procédé de préparation des co-cristaux d'agomélatine et d'acide *p*-toluènesulfonique selon l'une des revendications 1 à 3 **caractérisé en ce que** les deux constituants sont co-broyés.

6. Procédé de préparation des co-cristaux d'agomélatine et d'acide *p*-toluènesulfonique selon l'une des revendications 1 à 3 **caractérisé en ce que** les deux constituants sont mélangés au sein d'un solvant organique ou hydro-organique puis congelés et séchés à très basse température.

7. Procédé de préparation des co-cristaux d'agomélatine et d'acide *p*-toluènesulfonique selon l'une des revendications 1 à 3 **caractérisé en ce que** les poudres d'agomélatine et de l'acide considéré sont mélangées dans un mélangeur, puis le mélange est extrudé par extrusion bi-vis sans filière pour obtenir un grain solide directement en sortie d'extrudeuse.

8. Compositions pharmaceutiques contenant comme principe actif un des co-cristaux d'agomélatine et d'acide *p*-toluènesulfonique selon l'une des revendications 1 à 3, en combinaison avec un ou plusieurs véhicules inertes, non toxiques et pharmaceutiquement acceptables.

9. Utilisation de compositions pharmaceutiques selon la revendication 8 pour la fabrication de médicaments pour traiter les troubles du système mélatoninergique.

10. Utilisation de compositions pharmaceutiques selon la revendication 8 pour la fabrication de médicaments pour le traitement du stress, des troubles du sommeil, des troubles de l'anxiété et notamment du trouble anxiété généralisée, des troubles obsessionnels compulsifs, des troubles de l'humeur et notamment des troubles bipolaires, de la dépression majeure, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, de la douleur, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale ainsi que dans les dysfonctionnements sexuels, en tant qu'inhibiteurs de l'ovulation, d'immunomodulateurs et dans le traitement des cancers.

11. Co-cristaux d'agomélatine et d'acide *p*-toluènesulfonique de formule (I) selon l'une des revendications 1 à 3 pour le traitement des troubles du système mélatoninergique.

12. Co-cristaux d'agomélatine et d'acide *p*-toluènesulfonique de formule (I) selon l'une des revendications 1 à 3 pour le traitement du stress, des troubles du sommeil, des troubles de l'anxiété et notamment du trouble anxiété généralisée, des troubles obsessionnels compulsifs, des troubles de l'humeur et notamment des troubles bipolaires, de la dépression majeure, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, de la douleur, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale ainsi que dans les dysfonctionnements sexuels, en tant qu'inhibiteurs de l'ovulation, d'immunomodulateurs et dans le traitement des cancers.

## Patentansprüche

1. Co-kristalline Kristallformen von Agomelatin und *p*-Toluolsulfonsäure der Formel (I): 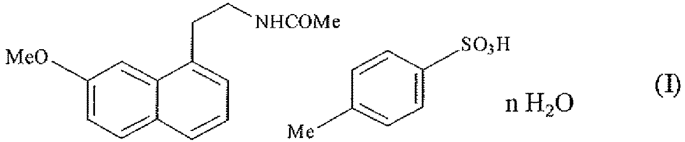 worin n 0 oder 1 beträgt.

2. Co-Kristall von Agomelatin und *p*-Toluolsulfonsäure-Monohydrat der Formel (I) nach Anspruch 1, **gekennzeichnet durch** das folgende Pulverröntgenbeugungsdiagramm, angegeben als Netzebenenabstand d, Bragg 2-Theta-Winkel (ausgedrückt in ° ± 0,2) und relative Intensität:
| 2-Theta (°) exp. | d (Å) exp. | Intensität (%) |
|---|---|---|
| 6,9631 | 12,6846 | 77,52 |
| 9,4831 | 9,31871 | 16,21 |
| 12,8823 | 6,86648 | 100 |
| 13,9527 | 6,34201 | 23,16 |
| 14,1761 | 6,24258 | 16,06 |
| 15,1817 | 5,83128 | 13,38 |
| 15,379 | 5,75689 | 25 |
| 15,5788 | 5,68351 | 46,24 |
| 16,7156 | 5,29947 | 94,46 |
| 17,2926 | 5,12391 | 37,98 |
| 18,4671 | 4,80058 | 92,47 |
| 18,6356 | 4,75756 | 22,34 |
| 19,199 | 4,6192 | 25,69 |
| 19,6747 | 4,50857 | 35,74 |
| 20,1398 | 4,4055 | 28,53 |
| 21,6248 | 4,1062 | 14,93 |
| 22,0586 | 4,02643 | 52,23 |
| 22,2859 | 3,98587 | 99,09 |
| 23,2175 | 3,82799 | 15,22 |
| 23,9607 | 3,71092 | 32,37 |
| 25,1733 | 3,53485 | 42,09 |
| 26,0152 | 3,42233 | 16,64 |
| 27,7148 | 3,2162 | 39,29 |
| 28,23 | 3,15866 | 11,25 |
| 28,4033 | 3,13979 | 16,33 |
einschließlich Formen, deren Beugungswinkel ± 0,2 ° betragen.

3. Co-Kristall von Agomelatin und *p*-Toluolsulfonsäure der Formel (I) nach Anspruch 1, **gekennzeichnet durch** sein folgendes Pulverröntgenbeugungsdiagramm angegeben als Netzebenenabstand d, Bragg 2-Theta-Winkel (ausgedrückt in ° ± 0,2) und relative Intensität:
| 2-Theta (°) exp. | d (Å) exp. | Intensität (%) |
|---|---|---|
| 11,2964 | 7,82664 | 21,53 |
| 11,6596 | 7,58367 | 20,45 |
| 13,4436 | 6,58103 | 61,31 |
| 15,2416 | 5,80848 | 18,42 |
| 16,0185 | 5,52847 | 30,89 |
| 17,3473 | 5,10789 | 41,39 |
| 17,8289 | 4,97096 | 54,3 |
| 18,2535 | 4,85629 | 100 |
| 20,4891 | 4,33118 | 19,84 |
| 20,6912 | 4,28932 | 45,12 |
| 20,9516 | 4,23659 | 36,73 |
| 21,3088 | 4,16638 | 14,93 |
| 22,2998 | 3,98342 | 33,92 |
| 23,129 | 3,84244 | 24,66 |
| 23,4107 | 3,79685 | 12,89 |
| 23,6474 | 3,75938 | 12,34 |
| 23,9983 | 3,7052 | 12,8 |
einschließlich Formen, deren Beugungswinkel ± 0,2 ° betragen.

4. Verfahren zur Herstellung von Co-Kristallen von Agomelatin und p-Toluolsulfonsäure gemäß einem der Ansprüche 1 bis 3, **dadurch gekennzeichnet, dass**:
- Agomelatin und *p*-Toluolsulfonsäure-Monohydrat in einem organischen oder wässrig-organischen Lösungsmittel in den gewünschten Verhältnissen vermischt werden;
- die erhaltene Lösung gerührt und gegebenenfalls auf eine Temperatur höchstens gleich der Siedetemperatur des gewählten Lösungsmittels erhitzt wird;
- das Medium unter Rühren abgekühlt wird und der Komplex natürlich ausfällt oder nach der Aufnahme in ein zweites Lösungsmittel ausfällt;
- der erhaltene Niederschlag abfiltriert und getrocknet wird;
- gegebenenfalls der Niederschlag unter Erhitzen getrocknet wird.

5. Verfahren zur Herstellung von Co-Kristallen von Agomelatin und p-Toluolsulfonsäure gemäß einem der Ansprüche 1 bis 3, **dadurch gekennzeichnet, dass** die beiden Bestandteile gemeinsam vermahlen werden.

6. Verfahren zur Herstellung von Co-Kristallen von Agomelatin und *p-*Toluolsulfonsäure gemäß einem der Ansprüche 1 bis 3, **dadurch gekennzeichnet, dass** die beiden Bestandteile in einem organischen oder wässrig-organischen Lösungsmittel vermischt und dann eingefroren und bei sehr niedriger Temperatur getrocknet werden.

7. Verfahren zur Herstellung von Co-Kristallen von Agomelatin und *p-*Toluolsulfonsäure gemäß einem der Ansprüche 1 bis 3, **dadurch gekennzeichnet, dass** die Pulver von Agomelatin und der in Rede stehenden Säure in einem Mischer vermischt werden und die Mischung dann mit einem Doppelschneckenextruder ohne Düse extrudiert wird unter Erhalt eines festen Korns direkt am Ausgang des Extruders.

8. Pharmazeutische Zubereitungen, enthaltend als Wirkstoff eines der Co-Kristalle von Agomelatin und *p*-Toluolsulfonsäure gemäß einem der Ansprüche 1 bis 3 in Kombination mit einem oder mehreren inerten, nichttoxischen und pharmazeutisch annehmbaren Hilfsstoffen.

9. Verwendung der pharmazeutischen Zubereitungen nach Anspruch 8 zur Herstellung von Arzneimitteln zur Behandlung von Störungen des melatoninergischen Systems.

10. Verwendung der pharmazeutischen Zubereitungen nach Anspruch 8 zur Herstellung von Arzneimitteln zur Behandlung von Stress, Schlafstörungen, Angststörungen, insbesondere allgemeine Angststörungen, kompulsiven Zwangsstörungen, Gemütsstörungen und insbesondere bipolaren Störungen, Major-Depression, saisonal bedingten Depressionen, kardiovaskulären Erkrankungen, Erkrankungen des Verdauungssystems, Schlaflosigkeit und Müdigkeit als Folge von Zeitverschiebungen, Schizophrenie, Panikanfällen, Melancholie, Appetitstörungen, Fettsucht, Schlaflosigkeit, Schmerzen, psychotischen Störungen, Epilepsie, Diabetes, der Parkinsonschen Krankheit, der senilen Demenz, verschiedenen Störungen, die mit dem normalen oder pathologischen Altern verknüpft sind, Migräne, Gedächtnisverlusten, der Alzheimerschen Krankheit sowie Gehirndurchblutungsstörungen sowie sexuellen Dysfunktionen, als Ovulationsinhibitoren, Immunomodulatoren und bei der Behandlung von Krebs.

11. Co-Kristalle von Agomelatin und *p*-Toluolsulfonsäure der Formel (I) nach einem der Ansprüche 1 bis 3 zur Behandlung von Störungen des melatoninergischen Systems.

12. Co-Kristalle von Agomelatin und *p*-Toluolsulfonsäure der Formel (I) nach einem der Ansprüche 1 bis 3 zur Behandlung von Stress, Schlafstörungen, Angststörungen, insbesondere allgemeine Angststörungen, kompulsiven Zwangsstörungen, Gemütsstörungen und insbesondere bipolaren Störungen, Major-Depression, saisonal bedingten Depressionen, kardiovaskulären Erkrankungen, Erkrankungen des Verdauungssystems, Schlaflosigkeit und Müdigkeit als Folge von Zeitverschiebungen, Schizophrenie, Panikanfällen, Melancholie, Appetitstörungen, Fettsucht, Schlaflosigkeit, Schmerzen, psychotischen Störungen, Epilepsie, Diabetes, der Parkinsonschen Krankheit, der senilen Demenz, verschiedenen Störungen, die mit dem normalen oder pathologischen Altern verknüpft sind, Migräne, Gedächtnisverlusten, der Alzheimerschen Krankheit sowie Gehirndurchblutungsstörungen sowie sexuellen Dysfunktionen, als Ovulationsinhibitoren, Immunomodulatoren und bei der Behandlung von Krebs.

## Claims

1. Crystalline forms of co-crystals of agomelatine and *p*-toluenesulphonic acid of formula (I): 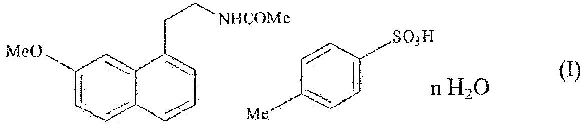 wherein n represents 0 or 1.

2. Co-crystal of agomelatine and *p*-toluenesulphonic acid monohydrate of formula (I) according to claim 1, **characterised by** its X-ray powder diffraction diagram expressed in terms of interplanar distance d, Bragg's angle 2 theta (expressed in °±0.2), and relative intensity, as follows:
| 2-Theta (°) exp. | d (Å) exp. | Intensity (%) |
|---|---|---|
| 6.9631 | 12.6846 | 77.52 |
| 9.4831 | 9.31871 | 16.21 |
| 12.8823 | 6.86648 | 100 |
| 13.9527 | 6.34201 | 23.16 |
| 14.1761 | 6.24258 | 16.06 |
| 15.1817 | 5.83128 | 13.38 |
| 15.379 | 5.75689 | 25 |
| 15.5788 | 5.68351 | 46.24 |
| 16.7156 | 5.29947 | 94.46 |
| 17.2926 | 5.12391 | 37.98 |
| 18.4671 | 4.80058 | 92.47 |
| 18.6356 | 4.75756 | 22.34 |
| 19.199 | 4.6192 | 25.69 |
| 19.6747 | 4.50857 | 35.74 |
| 20.1398 | 4.4055 | 28.53 |
| 21.6248 | 4.1062 | 14.93 |
| 22.0586 | 4.02643 | 52.23 |
| 22.2859 | 3.98587 | 99.09 |
| 23.2175 | 3.82799 | 15.22 |
| 23.9607 | 3.71092 | 32.37 |
| 25.1733 | 3.53485 | 42.09 |
| 26.0152 | 3.42233 | 16.64 |
| 27.7148 | 3.2162 | 39.29 |
| 28.23 | 3.15866 | 11.25 |
| 28.4033 | 3.13979 | 16.33 |
including the forms whose diffraction angles correspond to within ±0.2°.

3. Co-crystal of agomelatine and *p*-toluenesulphonic acid of formula (I) according to claim 1, **characterised by** its X-ray powder diffraction diagram expressed in terms of interplanar distance d, Bragg's angle 2 theta (expressed in °±0.2), and relative intensity, as follows:
| 2-Theta (°) exp. | d (Å) exp. | Intensity (%) |
|---|---|---|
| 11.2964 | 7.82664 | 21.53 |
| 11.6596 | 7.58367 | 20.45 |
| 13.4436 | 6.58103 | 61.31 |
| 15.2416 | 5.80848 | 18.42 |
| 16.0185 | 5.52847 | 30.89 |
| 17.3473 | 5.10789 | 41.39 |
| 17.8289 | 4.97096 | 54.3 |
| 18.2535 | 4.85629 | 100 |
| 20.4891 | 4.33118 | 19.84 |
| 20.6912 | 4.28932 | 45.12 |
| 20.9516 | 4.23659 | 36.73 |
| 21.3088 | 4.16638 | 14.93 |
| 22.2998 | 3.98342 | 33.92 |
| 23.129 | 3.84244 | 24.66 |
| 23.4107 | 3.79685 | 12.89 |
| 23.6474 | 3.75938 | 12.34 |
| 23.9983 | 3.7052 | 12.8 |
including the forms whose diffraction angles correspond to within ±0.2°.

4. Process for obtaining the co-crystals of agomelatine and *p*-toluenesulphonic acid according to any one of claims 1 to 3, **characterised in that**:
- agomelatine and *p*-toluenesulphonic acid monohydrate are mixed in an organic or aqueous-organic solvent in the desired proportions;
- the solution obtained is stirred and optionally heated at a temperature not greater than the boiling point of the chosen solvent;
- the mixture is cooled, with stirring, and the complex precipitates naturally or precipitates after being taken up in a second solvent;
- the precipitate obtained is filtered and dried;
- optionally, the precipitate is dried by heating.

5. Process for the preparation of the co-crystals of agomelatine and *p*-toluenesulphonic acid according to any one of claims 1 to 3, **characterised in that** the two constituents are co-ground.

6. Process for the preparation of the co-crystals of agomelatine and *p*-toluenesulphonic acid according to any one of claims 1 to 3, **characterised in that** the two constituents are mixed in an organic or aqueous-organic solvent and then frozen and dried at a very low temperature.

7. Process for the preparation of the co-crystals of agomelatine and *p*-toluenesulphonic acid according to any one of claims 1 to 3, **characterised in that** the powders of agomelatine and the acid in question are mixed in a mixer and the mixture is then extruded by twin-screw extrusion without a die in order to obtain a solid grain directly at the outlet of the extruder.

8. Pharmaceutical compositions comprising as active ingredient one of the co-crystals of agomelatine and *p*-toluenesulphonic acid according to any one of claims 1 to 3, in combination with one or more inert, non-toxic, pharmaceutically acceptable carriers.

9. Use of pharmaceutical compositions according to claim 8 in the manufacture of medicaments for treating disorders of the melatoninergic system.

10. Use of pharmaceutical compositions according to claim 8 in the manufacture of medicaments for treating stress, sleep disorders, anxiety disorders and especially generalised anxiety disorder, obsessive compulsive disorders, mood disorders and especially bipolar disorders, major depression, seasonal affective disorder, cardiovascular pathologies, pathologies of the digestive system, insomnia and fatigue due to jet-lag, schizophrenia, panic attacks, melancholia, appetite disorders, obesity, insomnia, pain, psychotic disorders, epilepsy, diabetes, Parkinson's disease, senile dementia, various disorders associated with normal or pathological ageing, migraine, memory loss, Alzheimer's disease, and also in cerebral circulation disorders, and also in sexual dysfunctions, as ovulation inhibitors and immunomodulators and in the treatment of cancers.

11. Co-crystals of agomelatine and *p*-toluenesulphonic acid of formula (I) according to any one of claims 1 to 3 for treating disorders of the melatoninergic system.

12. Co-crystals of agomelatine and *p*-toluenesulphonic acid of formula (I) according to any one of claims 1 to 3 for treating stress, sleep disorders, anxiety disorders and especially generalised anxiety disorder, obsessive compulsive disorders, mood disorders and especially bipolar disorders, major depression, seasonal affective disorder, cardiovascular pathologies, pathologies of the digestive system, insomnia and fatigue due to jet-lag, schizophrenia, panic attacks, melancholia, appetite disorders, obesity, insomnia, pain, psychotic disorders, epilepsy, diabetes, Parkinson's disease, senile dementia, various disorders associated with normal or pathological ageing, migraine, memory loss, Alzheimer's disease, and also in cerebral circulation disorders, and also in sexual dysfunctions, as ovulation inhibitors and immunomodulators and in the treatment of cancers.
